(19) Europäisches Patentamt
European Patent Office
Office européen des brevets

(11) **EP 3 845 220 A1**

(12) **EUROPEAN PATENT APPLICATION**
published in accordance with Art. 153(4) EPC

(43) Date of publication:
**07.07.2021 Bulletin 2021/27**

(21) Application number: **20816081.2**

(22) Date of filing: **30.10.2020**

(51) Int Cl.:
**A61K 9/16** (2006.01)     **A61K 47/38** (2006.01)
**A61K 31/715** (2006.01)    **A61K 31/717** (2006.01)
**A61P 7/04** (2006.01)

(86) International application number:
**PCT/KR2020/015000**

(87) International publication number:
**WO 2021/086097 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **31.10.2019 KR 20190138212**

(71) Applicant: **Samyang Biopharmaceuticals
Corporation
Seoul 03129 (KR)**

(72) Inventors:
• **KIM, Hyun Kyoon
Yongin-si
Gyeonggi-do 16833 (KR)**
• **HUH, Boem Kang
Seoul 04724 (KR)**
• **YOON, Hye Sung
Yongin-si
Gyeonggi-do 16827 (KR)**
• **KO, Suk Yen
Suwon-si
Gyeonggi-do 16505 (KR)**

(74) Representative: **Schrell, Andreas et al
Gleiss Große Schrell und Partner mbB
Patentanwälte Rechtsanwälte
Leitzstrasse 45
70469 Stuttgart (DE)**

(54) **POWDER-TYPE HEMOSTATIC COMPOSITION AND PREPARATION METHOD THEREFOR**

(57)     The present invention relates to a powder type hemostatic composition and method for preparing the same, and more specifically, to powder aggregate having porosity obtained by combining powder of biocompatible hemostatic material with a binder and a method for preparing the same, and a powder type hemostatic composition which comprises the powder aggregate and shows improved hemostatic performance as compared with simple powder hemostatic agents, and can be used to a large surface area for hemostasis, or to narrow, thin or other sites to which approach for hemostasis is difficult.

【FIGURE 1】

**Description**

**TECHNICAL FIELD**

The present invention relates to a powder type hemostatic composition and method for preparing the same, and more specifically, to powder aggregate having porosity obtained by combining powder of biocompatible hemostatic material with a binder and a method for preparing the same, and a powder type hemostatic composition which comprises the powder aggregate and shows improved hemostatic performance as compared with simple powder hemostatic agents, and can be used to a large surface area for hemostasis, or to narrow, thin or other sites to which approach for hemostasis is difficult.

**BACKGROUND ART**

Hemostasis (reduction of bleeding) is to pursue the patients' safety and convenience by minimizing blood loss, decreasing possibility of blood transfusion, shortening operation time, reducing postoperative complication, etc. due to blood transfusion, and eventually shortening hospitalization period. During surgical operation or procedure, bleeding can occur from large or small blood vessels in a large or small amount, and there are various hemostatic methods according to the bleeding amounts.

The commercially available hemostatic products at present use polysaccharides such as oxidized cellulose (OC), oxidized regenerated cellulose (ORC), methyl cellulose, ethyl cellulose, dextran, etc., natural polymers derived from animal or human such as collagen, gelatin, fibrin, thrombin, etc., or natural polymers derived from non-living body, and are prepared in fabric, non-woven fabric, sponge, sheet such as film, colloid or gel form.

However, it is difficult to apply hemostatic products in the above forms quickly and precisely to the bleeding site. The another problem is that they are difficult to apply to a large surface area for hemostasis, or to narrow, thin or other sites to which approach for hemostasis is difficult.

Various alternative hemostatic products have been suggested. For instance, US Patent No. 8709463 discloses a hemostatic product in sponge, patch or bead form prepared by chopping or shredding oxidized regenerated cellulose (ORC) fibers, making rod-shaped ORC fibers in about 35-4350 $\mu$m size by a method of cryo-milling or the like using liquid nitrogen, and mixing the fibers with a solution of water-soluble, water-swellable polysaccharides (sodium carboxy cellulose, etc.). In addition, US Patent No. 6060461 discloses that particles are prepared by crosslinking dextran (polysaccharide) and used as a hemostatic material. However, such alternative hemostatic products have disadvantages such as poor hemostatic effect due to low blood penetration rate when contacted with blood, and possible risk of wash out during surgical operation or procedure.

Therefore, medical teams and patients have continuously required a powder type hemostatic product which can be easily applied to a large surface area or to narrow, thin or other wound sites to which approach is difficult, can be easily used by medical teams, and can satisfy high blood absorption rate and good blood aggregation effect simultaneously.

**PROBLEMS TO BE SOLVED**

The purpose of the present invention is to provide powder aggregate which shows improved hemostatic performance as compared with simple powder hemostatic agents, and can be used to a large surface area for hemostasis, or to narrow, thin or other sites to which approach for hemostasis is difficult, a method for preparing the same, and a powder type hemostatic composition comprising the same.

**TECHNICAL MEANS**

In order to achieve the above purpose, the present invention provides powder aggregate for hemostatisis, wherein the powder aggregate is obtained by combining powder of biocompatible hemostatic material with a binder, and has a porosity ratio of from 1% to 50%.

A second aspect of the present invention provides a method for preparing powder aggregate for hemostatisis, comprising: spraying a binder solution to powder of biocompatible hemostatic material to form powder aggregate having a porosity ratio of from 1% to 50%.

A third aspect of the present invention provides a powder type hemostatic composition comprising the powder aggregate of the present invention.

**EFFECT OF THE INVENTION**

The powder aggregate for hemostatisis according to the present invention has a high porosity ratio and thereby a large surface area, and thus improves hemostatic effect through rapid blood absorption when contacted with blood. In addition, it absorbs body fluid and forms a gel quickly, and thereby acts as a physical barrier at the wound site sufficiently. Furthermore, it forms blood clot stably, and after finishing the hemostatic function, it is decomposed and absorbed into the body, and thereby foreign body reaction can be reduced. In particular, the powder aggregate for hemostatisis according to the present invention can be used very suitably to a large surface area for hemostasis, or to narrow, thin or other sites to which approach for hemostasis is difficult.

**BRIEF EXPLANATION OF THE DRAWINGS**

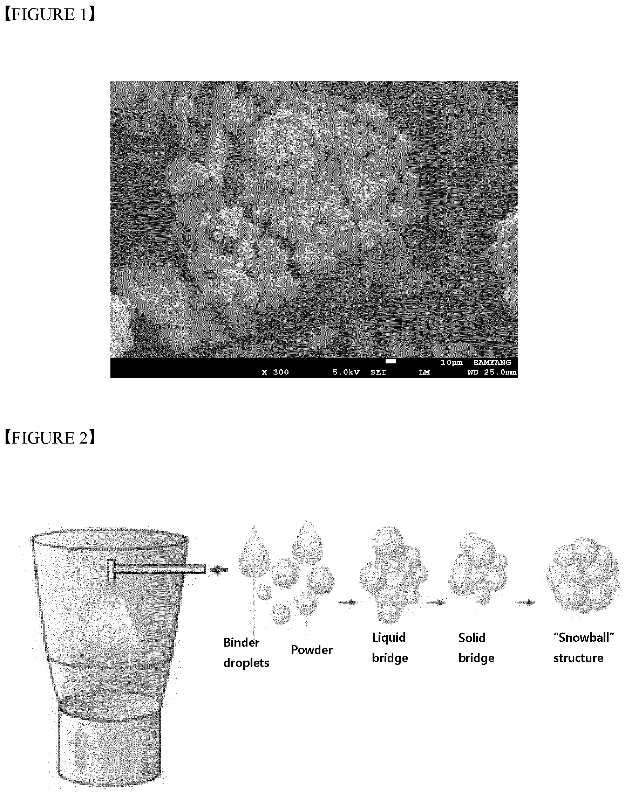

Fig. 1 is a Scanning Electron Microscope (SEM) photograph of porous ORC powder aggregate prepared according to an embodiment of the present invention.
Fig. 2 is a scheme for a procedure of preparing porous powder aggregate by using a fluidized bed granulator according to an embodiment of the present invention.

**CONCRETE MODE FOR CARRYING OUT THE INVENTION**

The present invention is explained in more detail below.
In the present invention, the biocompatible hemostatic material can be cellulose-based material, natural polymer or combination thereof, and more concretely, can be selected from oxidized celluloses and neutralized products thereof, oxidized regenerated celluloses and neutralized products thereof, polysaccharides (for example, methyl cellulose, ethyl cellulose, dextran, etc.), natural polymers derived from living body (for example, collagen, gelatin, fibrin, thrombin, chitosan-based polymers, chitin-based polymers, etc.), natural polymers derived from non-living body and combinations thereof.
The form of the biocompatible hemostatic material is not especially limited, and can be a fiber form or a sheet form, for example, but it is not limited thereto.
The biocompatible hemostatic material can be made into powder through mechanical pulverization, and there is no special limitation to the method or device for pulverization. Conventionally known means such as jet mill, cryo-mill, cutting mill, etc. can be used, and the pulverization can be conducted one time or several times.
In an embodiment, the powder aggregate for hemostatisis of the present invention obtained by combining powder of biocompatible hemostatic material with a binder has a porous structure with plural micro-channels, and its porosity ratio calculated by Mercury Porosimetry can be 1% to 50%. More concretely, the porosity ratio can be 5% or higher, 7% or higher, or 10% or higher, and 45% or lower, 40% or lower, or 35% or lower, but it is not limited thereto. If the porosity ratio of the powder aggregate is lower than 1%, the absorption ratio thereof decreases remarkably, and if the porosity ratio is higher than 50%, the size of the aggregate increases excessively and the flowability decreases, resulting in reduced convenience in use.
According to an example of the present invention, the powder aggregate for hemostatisis has a particle size with D50 of from 80 μm to 300 μm and D90 of from 200 μm to 700 μm.
D50 can be defined as the particle diameter of accumulated 50% based on volume by laser diffraction scattering particle size distribution measurement, and D90 means the particle diameter of accumulated 90% based on volume.
In an embodiment, the powder aggregate for hemostatisis of the present invention can have a particle size with D50 of from 100 μm to 300 μm and D90 of from 220 μm to 690 μm, and more concretely, a particle size with D50 of from 120 μm to 290 μm and D90 of from 220 μm to 690 μm, or a particle size with D50 of from 150 μm to 280 μm and D90 of from 240 μm to 670 μm.
The powder aggregate for hemostatisis of the present invention can have a D90 to D50 ratio (=D90/D50) of from 1.2 to 3.0. It is practically hard to obtain powder aggregate with D90/D50 of less than 1.2. In addition, with use of powder aggregate with D90/D50 of greater than 3.0, it is difficult to obtain powder aggregate having the target particle diameter, and removal of coarse particle becomes difficult.
According to an example of the present invention, the D90 to D50 ratio of the powder aggregate for hemostatisis of the present invention can be in a range of from 1.3 to 3.0, from 1.3 to 2.7, from 1.3 to 2.5, from 1.4 to 3.0, from 1.4 to 2.7, from 1.4 to 2.5, from 1.5 to 3.0, from 1.5 to 2.7, from 1.5 to 2.5, from 1.6 to 3.0, from 1.6 to 2.7, from 1.6 to 2.5, from 1.7 to 3.0, from 1.7 to 2.7, from 1.7 to 2.5, from 1.8 to 3.0, from 1.8 to 2.7, from 1.8 to 2.5, from 1.9 to 3.0, from 1.9 to 2.7, from 1.9 to 2.5, from 2.0 to 3.0, from 2.0 to 2.7, from 2.0 to 2.5, from 2.0 to 2.4, or from 2.0 to 2.3.
In the present invention, the binder can be a biocompatible material having adsorbability and viscosity, and more concretely, can be one or more selected from the group consisting of cellulose type binder material, polyvinylpolypyrrolidone, polyvinylalcohol (PVA), starch and polyethylene oxide (PEO). According to an embodiment of the present invention, the cellulose type binder material can be selected from salts of carboxymethyl cellulose, hydroxyethyl cellulose, hydroxypropyl methylcellulose and combinations thereof.

The binder can be applied to the powder of biocompatible hemostatic material, for example, in a form dissolved or dispersed in a solvent such as distilled water, alcohols, etc. The concentration of the binder solution can be, for example, 0.1 to 10% by weight, but it is not limited thereto.

In an embodiment, the binder solution can be sprayed to the powder of biocompatible hemostatic material to form the powder aggregate comprising these ingredients. According to an embodiment, the binder solution can be sprayed while fluidizing the powder of biocompatible hemostatic material by using fluidized bed granulation technique. In an embodiment, when the binder solution is sprayed, the spray pressure can be 0.1 to 5 bars, and the spray rate can be 1 to 30 ml/min, but it is not limited thereto.

The binder amount in the powder aggregate formed as above can be in a range of, for example, from 0.1 to 5% by weight, from 0.1 to 4% by weight, from 0.1 to 3% by weight, from 0.1 to 2.5% by weight, from 0.5 to 4% by weight, from 0.5 to 3% by weight, from 0.5 to 2.5% by weight, or from 0.5 to 2.0% by weight, but it is not limited thereto. If the binder amount in the powder aggregate is less than 0.1% by weight, the powder aggregate is not formed substantially. If the binder amount in the powder aggregate is greater than 5% by weight, the production ratio of powder aggregate having the target size decreases remarkably.

There is no special limitation to a method or device for sorting the powder aggregate formed as above according to the size. Conventionally known means such as vibration sieve, etc. can be used.

The powder aggregate sorted according to the size can be subjected then to a drying step and optionally a sterilizing step.

In an embodiment, the powder aggregate according to the present invention can have an average particle size of 500 $\mu$m or less, more concretely 400 $\mu$m or less, and even more concretely 300 $\mu$m or less, but it is not limited thereto. There is no special limitation to the lower limit thereof, and it can be, for example, 20 $\mu$m, 30 $\mu$m or 50 $\mu$m.

The powder aggregate of the present invention can be applied to the bleeding site by various methods, and there is no special limitation to the application method. For example, it can be spray-applied through a spray type applicator. In this case, considering the spray performance, the average size of the powder aggregate of the present invention can be 500 $\mu$m or less. If the size is greater than this, the size of the aggregate becomes greater than the spay nozzle diameter of the applicator and the spaying may not be conducted, and the spray performance may be lowered due to the problem in flowability.

In an embodiment, the powder aggregate according to the present invention can have a circularity of from 0.1 to 1.0, and more concretely, a circularity of from 0.2 to 0.9, from 0.3 to 0.9, from 0.3 to 0.8, from 0.3 to 0.7, from 0.4 to 0.9, from 0.4 to 0.8, or from 0.4 to 0.7, but it is not limited thereto.

An example of the present invention provides a method for preparing powder aggregate of biocompatible hemostatic material having porous structure by using wet aggregation technique. There is no special limitation to the kind of the wet aggregation technique, but according to an embodiment of the present invention, a method for preparing powder aggregate for hemostatisis, wherein the powder aggregate is formed by spraying the binder solution while fluidizing the powder of biocompatible hemostatic material by using fluidized bed granulation technique, can be provided.

In addition, an example of the present invention can provide a powder type hemostatic composition comprising the powder aggregate for hemostatisis.

The present invention will be explained below in more detail with reference to the following Examples. However, the Examples are only to illustrate the invention, and the scope of the present invention is not limited thereby in any manner.

**[Examples 1 to 7]**

_1. Preparation of biocompatible hemostatic material powder_

The first pulverization of an ORC hemostatic material in fabric form was conducted by using a cutting mill, and the first pulverization product was fed into a cryo-mill or a jet mill to prepare ORC powder in fine size. The prepared fine powder had a particle size distribution of D50 $\leq$ 50 $\mu$m and D90 $\leq$ 100 $\mu$m as measured by Dynamic Light Scattering (DLS).

_2. Preparation of powder aggregate and measurement of D50, D90 size distribution_

The ORC fine powder as prepared above was fed into a fluidized bed granulator and a fluidized layer was formed therein. Then, a binder solution was sprayed to the ORC powder under fluidization to prepare an ORC powder aggregate. The binder solutions used in preparing the powder aggregates of Examples 1 to 7, respectively, are shown in the following Table 1.

The amount of the binder contained in the ORC powder aggregate affects the size distribution of the ORC powder aggregate. The powder aggregates of Examples 1 to 7 were prepared to have a binder amount of average 0.5% by weight, 2% by weight, or 3% by weight in the finally prepared ORC powder aggregate. The D50, D90 size distributions of the generated powder aggregates were measured and are shown in the following Table 1.

A commercially available product, Surgicel powder (Ethicon, J&J), was used as Comparative Example, and the D50, D90 size distribution of powder aggregate of Comparative Example was measured and is shown in the following Table 1.

3. Measurement of porosity and absorption rate

For the powder aggregates of Examples 1 to 7, the porosity (%) and liquid absorption rate ($m^2/t$) were measured and are shown in the following Table 1. Comparative Example (Surgicel powder) had no porosity, and its absorption rate was measured and is shown in the following Table 1.

The absorption rate was measured by using Washburn absorption method ($m^2/t \propto \cos\theta$) disclosed in the publication [Journal of Colloid and Interface Science 346 (2010) 470-475, Laurence Galet et al.]. The liquid absorption amount and absorption rate of powder aggregate were measured, and on the basis thereof, the rate of blood penetration and the weight amount of blood coagulation could be determined indirectly.

$$cos\theta = \frac{m^2}{t} * C_w \qquad \left( C_w = \frac{\eta}{\rho^2 \sigma c} \right)$$

Slope: cos θ (contact angle θ)
m: Maximum absorption amount of liquid
t: Time for the maximum absorption
$C_w$: Treated as constant under the same experimental condition
η: Viscosity of liquid
$\rho^2$: Density of liquid
σ: Surface tension of liquid
c: Material constant of solid sample

[Table 1]

| Sample # | Binder type | Binder amount | Size of ORC aggregate | | | Porosity (%) | Absorption rate ($m^2/t$) |
| --- | --- | --- | --- | --- | --- | --- | --- |
| | | | D50 (μm) | D90 (μm) | D90/D50 | | |
| Comparative Example-Surgicel powder | - | - | 203 | 360 | 1.8 | - | 5±2 |
| Example 1 | CMC-Na | 0.5 wt% | 150 | 240 | 1.6 | 10% | 35±3 |
| Example 2 | CMC-Na | 2 wt% | 210 | 460 | 2.2 | 31% | 52±3 |
| Example 3 | CMC-Na | 3 wt% | 280 | 670 | 2.4 | 45% | 64±5 |
| Example 4 | CMC-Ca | 0.5 wt% | 130 | 230 | 1.8 | 12% | 28±4 |
| Example 5 | CMC-Ca | 2 wt% | 195 | 437 | 2.2 | 29% | 45±5 |
| Example 6 | HPMC | 0.5 wt% | 167 | 251 | 1.5 | 5% | 12±4 |
| Example 7 | HPMC | 2 wt% | 189 | 426 | 2.3 | 18% | 25±5 |

As shown in Table 1 above, it was confirmed that differently from Comparative Example, the powder aggregates of Examples 1 to 7 had a porosity of about 5% to 45% and remarkably improved liquid absorption rates. It was also confirmed that as the binder amount increased, D50, D90 values for the size of ORC aggregate increased and accordingly the absorption rate increased. Considering the case of spraying by using a spray applicator for the powder hemostatic aggregate, Examples 2, 5 and 7 had a suitable size distribution of ORC aggregate. For the above three (3) ORC aggregates, the blood coagulation amount was measured further.

4. Measurement of blood coagulation amount

For Comparative Example (Surgicel powder) and the ORC aggregates of Examples 2, 5 and 7, the blood coagulation amount was measured through animal experiments, and the results are shown in the following Table 2.

Concretely, the blood coagulation amount was measured with reference to the experimental method for hemostatic efficacy disclosed in the publication [ACS Biomater. Sci. Eng., 2017, 3 (12), pp 3675-3686, Absorbable Hemostatic Aggregates, Allen Y. Wang et al.]. In a test tube, 1 ml of whole blood of rat was put and 100 mg of each sample was added thereto, and after 2 minutes, the weight of blood coagulation as generated was measured.

[Table 2]

| Sample # | Binder type | Binder amount | Size of ORC aggregate | | Blood coagulation amount (mg) |
|---|---|---|---|---|---|
| | | | D50 ($\mu$m) | D90 ($\mu$m) | |
| Comparative Example-Surgicel | - | | 203 | 360 | 150$\pm$15 |
| Example 2 | CMC-Na | 2 wt% | 210 | 460 | 450$\pm$45 |
| Example 5 | CMC-Ca | 2 wt% | 195 | 437 | 405$\pm$53 |
| Example 7 | HPMC | 2 wt% | 189 | 426 | 243$\pm$31 |

As shown in Table 2 above, it was confirmed that Examples 2, 5 and 7 of the present invention showed remarkably increased blood coagulation amount as compared with Comparative Example (Surgicel powder), and accordingly provided remarkably better hemostatic efficacy.

**Claims**

1. Powder aggregate for hemostatisis, wherein the powder aggregate is obtained by combining powder of biocompatible hemostatic material with a binder, and has a porosity ratio of from 1% to 50%.

2. The powder aggregate for hemostatisis of Claim 1, which has a particle size with D50 of from 80 $\mu$m to 300 $\mu$m and D90 of from 200 $\mu$m to 700 $\mu$m.

3. The powder aggregate for hemostatisis of Claim 1, wherein the biocompatible hemostatic material is selected from oxidized celluloses and neutralized products thereof, oxidized regenerated celluloses and neutralized products thereof, polysaccharides, natural polymers derived from living body, natural polymers derived from non-living body and combinations thereof.

4. The powder aggregate for hemostatisis of Claim 1, wherein the binder is one or more selected from the group consisting of cellulose type binder material, polyvinylpyrrolidone, polyvinylalcohol (PVA), starch and polyethylene oxide (PEO).

5. The powder aggregate for hemostatisis of Claim 4, wherein the cellulose type binder material is selected from salts of carboxymethyl cellulose, hydroxyethyl cellulose, hydroxypropyl methylcellulose and combinations thereof.

6. The powder aggregate for hemostatisis of Claim 1, wherein the binder amount in the powder aggregate is in a range of from 0.1 to 5% by weight.

7. A method for preparing powder aggregate for hemostatisis, comprising: spraying a binder solution to powder of biocompatible hemostatic material to form powder aggregate having a porosity ratio of from 1% to 50%.

8. The method for preparing powder aggregate for hemostatisis of Claim 7, wherein the powder aggregate is formed by spraying the binder solution to the powder of biocompatible hemostatic material while fluidizing the powder of biocompatible hemostatic material by using fluidized bed granulation technique.

9. A powder type hemostatic composition comprising the powder aggregate for hemostatisis of any one of Claims 1 to 6.

【FIGURE 1】

【FIGURE 2】

Binder droplets · Powder · Liquid bridge · Solid bridge · "Snowball" structure

## INTERNATIONAL SEARCH REPORT

| International application No. |
|---|
| **PCT/KR2020/015000** |

### A.   CLASSIFICATION OF SUBJECT MATTER

**A61K 9/16**(2006.01)i; **A61K 47/38**(2006.01)i; **A61K 31/715**(2006.01)i; **A61K 31/717**(2006.01)i; **A61P 7/04**(2006.01)i

According to International Patent Classification (IPC) or to both national classification and IPC

### B.   FIELDS SEARCHED

Minimum documentation searched (classification system followed by classification symbols)

A61K 9/16(2006.01); A61K 38/36(2006.01); A61K 47/02(2006.01); A61K 47/38(2006.01); A61L 15/22(2006.01); A61L 15/44(2006.01); A61L 24/04(2006.01); A61L 26/00(2006.01); A61P 17/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체적합(biocompatible), 지혈(hemostasis), 분말(powder), 바인더(binder), 다공 (porous), 표면적(surface)

### C.   DOCUMENTS CONSIDERED TO BE RELEVANT

| Category* | Citation of document, with indication, where appropriate, of the relevant passages | Relevant to claim No. |
|---|---|---|
| X | JP 2012-527924 A (PROFIBRIX B.V.) 12 November 2012 (2012-11-12)<br>See claims 1, 3 and 9, and paragraphs [0007], [0009], [0017], [0019], [0024]-[0028], [0034]-[0036], [0041]-[0042], [0047], [0050] and [0059]-[0061]. | 1-9 |
| A | KR 10-2015-0015517 A (ETHICON INC) 10 February 2015 (2015-02-10)<br>See entire document. | 1-9 |
| A | KR 10-2014-0074990 A (BAXTER INTERNATIONAL INC. et al.) 18 June 2014 (2014-06-18)<br>See entire document. | 1-9 |
| A | KR 10-2012-0125465 A (PROFIBRIX B.V.) 15 November 2012 (2012-11-15)<br>See entire document. | 1-9 |
| A | KR 10-2015-0075516 A (SAMYANG BIOPHARMACEUTICALS CORPORATION) 06 July 2015 (2015-07-06)<br>See entire document. | 1-9 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 February 2021** | **03 February 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2020/015000**

| Patent document cited in search report | | | Publication date (day/month/year) | Patent family member(s) | | | Publication date (day/month/year) |
|---|---|---|---|---|---|---|---|
| JP | 2012-527924 | A | 12 November 2012 | CN | 102448443 | A | 09 May 2012 |
| | | | | EP | 2435028 | A2 | 04 April 2012 |
| | | | | EP | 2435028 | B1 | 31 August 2016 |
| | | | | JP | 5820807 | B2 | 24 November 2015 |
| | | | | US | 2012-0070477 | A1 | 22 March 2012 |
| | | | | US | 9119897 | B2 | 01 September 2015 |
| | | | | WO | 2010-136588 | A2 | 02 December 2010 |
| | | | | WO | 2010-136588 | A3 | 21 April 2011 |
| KR | 10-2015-0015517 | A | 10 February 2015 | CN | 104321085 | A | 28 January 2015 |
| | | | | CN | 104321085 | B | 23 June 2017 |
| | | | | EP | 2854882 | A1 | 08 April 2015 |
| | | | | EP | 2854882 | B1 | 31 October 2018 |
| | | | | EP | 3466455 | A1 | 10 April 2019 |
| | | | | JP | 2015-517388 | A | 22 June 2015 |
| | | | | JP | 6165849 | B2 | 19 July 2017 |
| | | | | KR | 10-2020-0085904 | A | 15 July 2020 |
| | | | | KR | 10-2131601 | B1 | 09 July 2020 |
| | | | | US | 2013-0316974 | A1 | 28 November 2013 |
| | | | | US | 2014-0329769 | A1 | 06 November 2014 |
| | | | | US | 8815832 | B2 | 26 August 2014 |
| | | | | US | 9539358 | B2 | 10 January 2017 |
| | | | | WO | 2013-177242 | A1 | 28 November 2013 |
| KR | 10-2014-0074990 | A | 18 June 2014 | CN | 103957948 | A | 30 July 2014 |
| | | | | CN | 103957948 | B | 26 October 2016 |
| | | | | EP | 2766058 | A2 | 20 August 2014 |
| | | | | JP | 2012-110512 | A | 14 June 2012 |
| | | | | JP | 2014-528336 | A | 27 October 2014 |
| | | | | JP | 2017-124316 | A | 20 July 2017 |
| | | | | JP | 6195568 | B2 | 13 September 2017 |
| | | | | KR | 10-1700990 | B1 | 31 January 2017 |
| | | | | KR | 10-2012-0064705 | A | 19 June 2012 |
| | | | | KR | 10-2135484 | B1 | 20 July 2020 |
| | | | | US | 10322170 | B2 | 18 June 2019 |
| | | | | US | 2013-0090291 | A1 | 11 April 2013 |
| | | | | US | 2013-0096082 | A1 | 18 April 2013 |
| | | | | US | 2016-0271228 | A1 | 22 September 2016 |
| | | | | WO | 2013-053749 | A2 | 18 April 2013 |
| | | | | WO | 2013-053749 | A3 | 14 November 2013 |
| | | | | WO | 2013-053755 | A2 | 18 April 2013 |
| | | | | WO | 2013-053755 | A3 | 14 November 2013 |
| KR | 10-2012-0125465 | A | 15 November 2012 | CN | 102724968 | A | 10 October 2012 |
| | | | | EP | 2521538 | A1 | 14 November 2012 |
| | | | | EP | 2521538 | B1 | 24 October 2018 |
| | | | | JP | 2013-516444 | A | 13 May 2013 |
| | | | | JP | 2016-074673 | A | 12 May 2016 |
| | | | | US | 2012-0315305 | A1 | 13 December 2012 |
| | | | | US | 8846105 | B2 | 30 September 2014 |
| | | | | WO | 2011-083154 | A1 | 14 July 2011 |
| KR | 10-2015-0075516 | A | 06 July 2015 | KR | 10-1588633 | B1 | 26 January 2016 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8709463 B

- US 6060461 A

**Non-patent literature cited in the description**

- **LAURENCE GALET.** *Journal of Colloid and Interface Science,* 2010, vol. 346, 470-475

- **ALLEN Y. WANG.** Absorbable Hemostatic Aggregates. *ACS Biomater. Sci. Eng.,* 2017, vol. 3 (12), 3675-3686